# EUROPÄISCHE PATENTSCHRIFT

(11) **EP 0 899 258 B1**
(45) Veröffentlichungstag und Bekanntmachung des Hinweises auf die Patenterteilung: **25.09.2002**
(21) Anmeldenummer: 98112125.4
(22) Anmeldetag: 01.07.1998
(51) Int. Cl.: C07C 319/26, C07C 323/22, C07C 45/86, C07C 47/02, C07F 9/09

(54) **Aliphatische Alkanale mit verbesserter Lagerstabilität und Verfahren zur Verbesserung der Lagerstabilität**
Aliphatic alkanals with improved storage stability and process for improving their stability
Aldéhydes aliphatiques avec stabilité de stockage améliorée et procédé pour l'amelioration de la stabilité

(30) Priorität: 14.08.1997 DE 19735332
(43) Veröffentlichungstag der Anmeldung: 03.03.1999
(73) Patentinhaber: Degussa AG, 40474 Düsseldorf (DE)
(72) Erfinder: Hasselbach, Hans Joachim Dr., 63571 Gelnhausen (DE); Huthmacher, Klaus Dr., 63571 Gelnhausen (DE); Kelm, Katja, 63796 Kahl (DE); Weigel, Horst, 63517 Rodenbach (DE)

(56) Entgegenhaltungen:
- WO-A-93/13059
- US-A- 4 289 912
- US-A- 4 546 205
- DATABASE WPI Section Ch, Week 9252 Derwent Publications Ltd., London, GB; Class D23, AN 92-431227 XP002087426 KARPUKHINA, G.V. ET AL: & SU 1 705 273 A (CHEM PHYS INST) , 15. Januar 1992

## Beschreibung

Die vorliegende Erfindung betrifft lagerstabile Zusammensetzungen, enthaltend aliphatische Alkanale mit einem Wassergehalt von > 300 ppm und ein Verfahren zur Verbesserung der Lagerstabilität dieser aliphatischen Alkanale, insbesondere von substituierten, schwefelhaltigen Alkanalen. Es ist bekannt, daß Verbindungen dieser Stoffklasse sehr reaktionsfreudig sind, und daß schon bei der Lagerung Veränderungen eintreten.

Durch die Bildung von Oligomeren, Polymeren und Kondensationsprodukten sowie Eliminierungsreaktionen und Oxydation und den Folgereaktionen der gebildeten Folgeprodukte entsteht eine Vielzahl von unerwünschten Verunreinigungen.

Ziel unserer Arbeiten war es, durch geeignete Zusätze in geringer Dosierung die Bildung der unerwünschten höhermolekularen Folgeprodukte zu verlangsamen.

Es ist bekannt, daß die Nebenproduktbildung bei der Lagerung von Alkanalen durch alkalische oder basische Bedingungen beeinflußt werden kann. In saurem Umfeld wird die Bildung von Oligomeren, Polymeren und vor allem cyclischen Trimeren (Trioxanen) begünstigt, in alkalischem Umfeld wird die Aldolkondensation begünstigt. So benutzt man gemäß DE-OS 2 905 267 in einem Verfahren zur Stabilisierung von Aldehyden vor cyclischer Trimerisation, Polymerisation und Autokondensation Triethanolamin oder N,N-Dimethylethanolamin und erzeugt so ein alkalisches Umfeld. Ebenso werden in den japanischen Patenten JP 72 321, 963 und JP 49116017 alkylierte Aniline zum gleichen Zweck eingesetzt. Die US-PS 4,546,205 betrifft die Verwendung eines Gemischs aus einem Pyridin und einer Phenolkomponente.

Die genannten Stabilisatoren erzeugen ein basisches Umfeld und verringern die Geschwindigkeit der sauer katalysierten Reaktionen. Die Stabilisatoren sind teilweise toxisch und werden zum Teil in hoher Dosierung, vorzugsweise bis zu 0,2 % eingesetzt. Aufgrund der Siedepunkte und der Produkteigenschaften ist eine destillative Abtrennung oft nicht möglich.

Aus der US-PS 4 289 912 ist die Stabilisierung von konzentrierten wässrigen Lösungen von Paraformaldehyd bekannt, mit der das Ausfällen von festen Polymeren verhindert werden soll. Für diesen Zweck werden verschiedene Verbindungen wie zum Beispiel Polyacrylsäure, Weinsäure, Äpfelsäure, Zitronensäure oder Ethylendiamintetraessigsäure eingesetzt.

Die SU 1705273 A1 (Derwent XP-002087426) betrifft die Stabilisierung von Phenylacetaldehyd. Als Stabilisatoren werden 3-Komponenten-Gemische eingesetzt, die zum Beispiel aus Diphenylamin, Butoxytoluol und einer Hydroxysäure bestehen.

In der PCT WO 93/13059 wird die Stabilisierung von schwefel-substituierten Alkanalen mit einer Mischung aus einer Aminkomponente zusammen mit einer sauerstoffbindenden Komponente, wie Phenolen, sauren oder ungesättigten Antioxydantien wie Ascorbinsäure oder beta-Carotin beschrieben. Bei diesem Verfahren wird jedoch darauf verwiesen, daß es nur wirksam ist, wenn der Wassergehalt des Alkanals unter 300 ppm liegt, vorzugsweise unter 100 ppm.

Unter Produktionsbedingungen ist diese Vorgabe nur mit erheblichem Aufwand einzuhalten. Zur Vermeidung der Autoxydation ist eine Überlagerung mit Stickstoff üblich. Keines der beschriebenen Verfahren zur Stabilisierung von Alkanalen erfüllt die Forderung, nach der die Stabilisierung mit untoxischen Stoffen in geringer Dosierung erfolgt und ein Wassergehalt bis zu 2 % toleriert werden kann.

Gegenstand der Erfindung sind verbesserte lagerstabile Zusammensetzungen, enthaltend aliphatische Alkanale der allgemeinen Formel 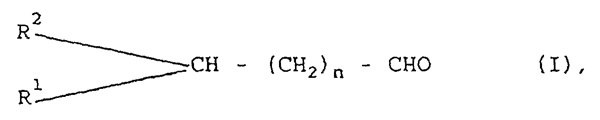 in der bedeuten:
- R¹, R² :: gleich oder verschieden: Wasserstoff, Alkyl mit 1 bis 3 C-Atomen oder einmal eine Alkylthiogruppe mit ein oder 2 C-Atomen,
- n :: eine ganze Zahl von 0 bis 4,
und einem Wassergehalt > 300 ppm und eine organische, gesättigte, Metallkomplexe bildende Säure ohne antioxidierende Wirkung. Diese Säuren brauchen nicht die im Stand der Technik geforderte antioxydierende Wirkung zu zeigen. Weitere Ausführungsformen finden sich in den abhängigen Ansprüchen. Besonderer Gegenstand des ebenfalls beanspruchten Verfahrens der Stabilisierung ist das Methylmercaptopropanal (MMP).

Es wurde gefunden, daß durch Zusatz der sauren verbindungen nicht nur die Aldolreaktion zurückgedrängt wird, sondern daß vor allem auch die im Sauren begünstigte Bildung der cyclischen Trimeren wesentlich verzögert wird. Der in technischen Alkanalen übliche Wassergehalt bis zu 2 Gew.-% beeinträchtigt, insbesondere bei Methylmercaptopropanal die Stabilisierung nicht.

Bei den genannten sauren Verbindungen handelt es sich um solche Verbindungen, die aufgrund ihrer Struktur in der Lage sind, Metalle zu komplexieren, insbesondere Hydroxydicarbonsäuren wie z. B. Zitronensäure, Weinsäure oder Äpfelsäure, insbesondere auch Aminotrismethylenphosphonsäure (ATMP).

Im Falle der Weinsäure besteht in der Wirkung kein Unterschied zwischen chiraler Weinsäure und dem Racemat, jedoch ist die chirale Form aufgrund der besseren Löslichkeit bevorzugt, wobei wiederum aufgrund der Verfügbarkeit der L-Weinsäure der Vorzug gegeben wird.

Die Säuren können in fester Form, aber auch als Lösung z. B. in Wasser oder einem niederen Alkohol zudosiert werden. Die Zugabe in gelöster Form hat den Vorteil, daß eine Homogenisierung rasch erfolgt, jedoch ist Art der Zugabe so zu wählen, daß sich keine zweite Phase bildet. Für die Wirkung als Stabilisator ist die Form der Zugabe ohne Bedeutung.

Die stabilisierende Wirkung hält bis zu einer Temperatur von mindestens 50 bis 100°C, bevorzugt bis 60°C, an.

Die Proben werden bevorzugt mit Stickstoff überlagert.
Die wirksame Menge liegt bei 25 bis 1000 mg Säure/kg Alkanal, vorzugsweise werden 40 bis 600 mg/kg Alkanal eingesetzt.

Die Wirkung, insbesondere der Weinsäure, kann durch Zusatz eines Alkanolamins, (C₁-C₃-Alkanol), insbesondere von Trialkonolaminen insofern verbessert werden, daß die Stabilisatormenge bei gleicher Wirkung deutlich verringert werden kann. Die Menge an Alkanolaminen, bevorzugt an Trialkanolaminen, insbesondere Triethanolamin,in dieser Mischung liegt im Bereich von 20 bis 80 %, vorzugsweise von 40 bis 70 Gew.-%, bezogen auf die Säure, insbesondere Weinsäure.

Von dieser Mischung, insbesondere einer Mischung aus Weinsäure und Triethanolamin reicht eine Menge von 50 bis 150 mg/kg Alkanal, um die Lagerstabilität nachhaltig zu verbessern. Die Zugabe der Mischung erfolgt auch hier vorteilhaft als konzentrierte wäßrige Lösung der beiden Komponenten. Auch durch den Zusatz von Aminotrismethylenphosphonsäure (ATMP) wird die Bildung von Kondensations- und Oligomerisationsprodukten deutlich verlangsamt. Hiervon ist eine Dosierung von 5 bis 20 mg/kg Alkanal ausreichend. Höhere Dosierungen (100 mg/kg) führen am Beispiel des Methylmercaptopropanals zu Gelbfärbung.

Die nachfolgenden Beispiele sollen die Erfindung näher veranschaulichen ohne einschränkend zu wirken. Die Geschwindigkeit der unerwünschten Reaktionen ist bei niedriger Temperatur langsam. Um den Einfluß der Stabilisatoren zu verdeutlichen, haben wir Temperaturen oberhalb einer normalen Lagertemperatur gewählt.

Zur Beurteilung der Stabilisatorwirkung wurden jeweils aus der gleichen Charge unbehandelte Blindproben und Proben mit Stabilisatorzusatz bei isothermen Bedingungen gelagert und nach mehreren Wochen analysiert.

Die Summe der Oligomeren, Polymeren und Polykondensationsprodukte wurden anhand des Destillationsrückstandes unter standardisierten Bedingungen ermittelt. Die Bestimmung des cyclischen Trimeren erfolgte durch 1H-NMR-Spektroskopie in der Originalprobe.

### Beispiel 1

500 g Methylmercaptopropanal (MMP) mit einem Wassergehalt von 2,0 G%, werden in einer Glasflasche mit 500 mg einer 50 %igen wäßrigen Lösung von L-Weinsäure gemischt und zusammen mit einer Blindprobe aus der gleichen Produktionscharge in einem Thermostaten bei 30°C gelagert. Nach 50 und 70 Tagen werden von der Probe und der Blindprobe jeweils 100 g unter Standardbedingungen
(15 mbar, Bad bis 140°C) destilliert und der Rückstand bestimmt.

| Rückstand in G%: | | |
|---|---|---|
| **Lagerdauer** | **Blindprobe** | **Probe** |
| 50 Tage | 11,0 | 3,4 |
| 70 Tage | 28,3 | 5,0 |

### Beispiel 2

500 g Methylmercaptopropanal (MMP) mit einem Wassergehalt von 1,8 G% werden in einem Glasgefäß mit 250 mg L-Weinsäure gerührt, bis der Feststoff gelöst ist. Durch Einleiten von Argon wird die Luft verdrängt. Eine Blindprobe aus der gleichen Charge wird ebenso behandelt. Die Lagerung erfolgt bei 50°C. Nach jeweils 20, 50 und 70 Tagen erfolgt eine Rückstandsbestimmung.

| Rückstand in G%: | | |
|---|---|---|
| **Lagerdauer** | **Blindprobe** | **Probe** |
| 20 Tage | 9,3 | 2,0 |
| 50 Tage | 22,3 | 3,1 |
| 70 Tage | 33,6 | 4,3 |

Die Blindprobe enthält nach 20 Tagen 7,0 % trimeres MMP, bei der stabilisierten Probe liegt der Trimerengehalt nach 70 Tagen noch unter 1 %

### Beispiel 3

Als Stabilisator wird eine wäßrige Lösung von 40 G% L-Weinsäure und 20 G% Triethanolamin eingesetzt. 500 g Methylmercaptopropanal (MMP) werden 63 mg der Stabilisatorlösung vermischt und zusammen mit einer Blindprobe aus derselben Charge in einem Thermostaten bei 30°C gelagert.

| Rückstand in M%: | | |
|---|---|---|
| **Lagerdauer** | **Blindprobe** | **Probe** |
| 20 Tage | 1,5 | 0,9 |
| 50 Tage | 16,0 | 1,6 |

### Beispiel 4

Der Versuch von Beispiel 3 wird mit dem Alkanal derselben Charge wiederholt, jedoch werden die Proben bei 50°C gelagert.

| Rückstand in G% | | |
|---|---|---|
| **Lagerdauer** | **Blindprobe** | **Probe** |
| 20 Tage | 2,5 | 1,7 |
| 50 Tage | 18,9 | 4,5 |

### Beispiel 5

Vergleichsbeispiel. 500 g Methylmercaptopropanal (MMP) mit einem Wassergehalt von 1,8 G% werden in einem Glasgefäß mit 450 mg Triethanolamin und 50 mg Ascorbinsäure versehen und es wird solange gerührt, bis eine klare Lösung entsteht. Die Probe wird zusammen mit einer Blindprobe bei 50°C gelagert. Nach 20 und 50 Tagen wird der Destillationsrückstand bestimmt.

| Rückstand in G% | | |
|---|---|---|
| **Lagerdauer** | **Blindprobe** | **Probe** |
| 20 Tage | 2,5 | 8,9 |
| 50 Tage | 22,3 | 20,0 |

Nach 50 Tagen enthält die Blindprobe 16,4 % Trimeres, die behandelte Probe < 1 %.

### Beispiel 6

500 g Methylmercaptopropanal (MMP) werden mit 20 mg einer 50 %igen Aminotrismethylenphosphonsäure-Lösung (ATMP) vermischt und diese Probe wird zusammen mit einer Blindprobe derselben Charge bei 50°C gelagert.

| Rückstand in G% | | |
|---|---|---|
| **Lagerdauer** | **Blindprobe** | **Probe** |
| 20 Tage | 3,8 | 3,1 |
| 50 Tage | 27,6 | 5,2 |

### Beispiel 7

In 500 g Isobutyraldehyd (z. Synthese, Fa. Merck, Darmstadt) werden 500 mg einer 50 %igen L-Weinsäurelösung gelöst und die Probe zusammen mit einer Blindprobe der gleichen Charge bei 30°C gelagert.

| Rückstand in G%: | | |
|---|---|---|
| **Lagerdauer** | **Blindprobe** | **Probe** |
| 21 Tage | 1,2 | 1,0 |
| 94 Tage | 12,4 | 4,1 |

### Beispiel 8

Von 6 Stahlkontainern mit je 20 t Methylmercaptopropanal (MMP) werden die Container 1 bis 3 mit je 10 kg L-Weinsäure stabilisiert. Alle Container bleiben im Freien stehen und der Inhalt wird nach 38 Tagen analysiert.

Destillationsrückstand in G%. Bei Beginn der Lagerung beträgt der Rückstand 0,53 %.

| Rückstand in G%: | | |
|---|---|---|
| Container | Rückstand | Zunahme |
| 1, stabilisiert | 1,25 | 0,72 |
| 2, stabilisiert | 1,02 | 0,49 |
| 3, stabilisiert | 1,17 | 0,64 |
| 4, unstabilisiert | 1,91 | 1,38 |
| 5, unstabilisiert | 2,31 | 1,78 |
| 6 unstabilisiert | 1,79 | 1,26 |

Daraus errechnet sich eine mittlere Zunahme von 0,62 % bei der stabilisierten Ware und 1,47 % bei der unstabilisierten Ware.

## Patentansprüche

1. Zusammensetzungen, enthaltend aliphatische Alkanale der allgemeinen Formel 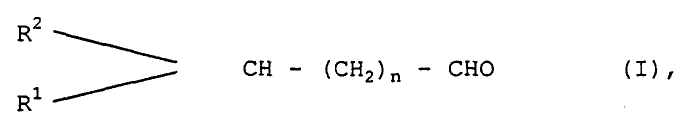 in der bedeuten:
R¹, R² : gleich oder verschieden: Wasserstoff, Alkyl mit 1 bis 3 C-Atomen oder einmal eine Alkylthiogruppe mit ein oder 2 C-Atomen,
n : eine ganze Zahl von 0 bis 4,
einen Wassergehalt bis 2 Gew.-% und eine organische, gesättigte, Metallkomplexe bildende Säure ohne antioxidierende Wirkung, die eine verbesserte Lagerstabilität zeigen.

2. Zusammensetzungen, gemäß Anspruch 1,
**dadurch gekennzeichnet,**
**daß** sie Säure in einer Menge von 0,004 bis bis 0,1 Gew.-%, bezogen auf die Menge des Alkanals, enthalten.

3. Zusammensetzungen, gemäß den Ansprüchen 1 und 2,
**dadurch gekennzeichnet,**
**daß** sie eine Hydroxydicarbonsäure enthalten.

4. Zusammensetzungen, gemäß dem Anspruch 3,
**dadurch gekennzeichnet,**
**daß** sie Weinsäure in chiraler oder racemischer Form enthalten.

5. Zusammensetzungen, gemäß den Ansprüchen 1 und 2,
**dadurch gekennzeichnet,**
**daß** sie Aminotrismethylenphosponsäure enthalten.

6. Zusammensetzungen, gemäß den Ansprüchen 1 bis 5,
**dadurch gekennzeichnet,**
**daß** sie zusätzlich Alkanolamine oder Trialkanolamine enthalten.

7. Zusammensetzungen, gemäß Anspruch 6,
**dadurch gekennzeichnet,**
**daß** sie ein Trialkanolamin enthalten, wobei Alkyl einem C₁ bis C₃-Alkylrest enspricht.

8. Zusammensetzungen, gemäß den Ansprüchen 6 und 7,
**dadurch gekennzeichnet,**
**daß** sie ein Trialkanolamin in einer Menge von 20 bis 80 Gew.-%, bezogen auf die organische Säure, enthalten.

9. Verfahren zur Verbesserung der Lagerstabilität von aliphatischen Alkanalen der allgemeinen Formel (I) mit einem wassergehalt > 300 ppm,
**dadurch gekennzeichnet,**
**daß** man diesen eine organische, gesättigte, Metallkomplexe bildende Säure ohne antioxidierende Wirkung, gegebenenfalls in Form einer wäßrigen oder alkoholischen Lösung zusetzt.

10. Verfahren gemäß Anspruch 9,
**dadurch gekennzeichnet,**
**daß** man die Säure in einer Menge von 0,04 bis 0,1 Gew.-%, bezogen auf die Menge des Alkanals, zusetzt.

11. Verfahren gemäß den Ansprüchen 9 und 10,
**dadurch gekennzeichnet,**
**daß** man eine Hydroxydicarbonsäure zusetzt.

12. Verfahren gemäß Anspruch 11,
**dadurch gekennzeichnet,**
**daß** man Weinsäure in chiraler oder racemischer Form zusetzt.

13. Verfahren gemäß den Ansprüchen 9 und 10,
**dadurch gekennzeichnet,**
**daß** man Aminotrismethylenphosphonsäure zusetzt.

14. Verfahren gemäß den Ansprüchen 9 bis 12,
**dadurch gekennzeichnet,**
**daß** man zusätzlich ein Alkanolamin oder Trialkanolamin zusetzt.

15. Verfahren gemäß Anspruch 14,
**dadurch gekennzeichnet,**
**daß** man Trialkanolamine in einer Menge von 20 bis 80 Gew.-%, bezogen auf die organische Säure gemäß Anspruchl, einsetzt.

16. Verfahren gemäß den Ansprüchen 14 und 15,
**dadurch gekennzeichnet,**
**daß** man das Alkanolamin oder Trialkanolamin in Kombination mit 0,005 bis 0,015 Gew.-% der Säure, bezogen auf das Alkanal, einsetzt.

## Claims

1. Compositions containing aliphatic alkanals of the general formula 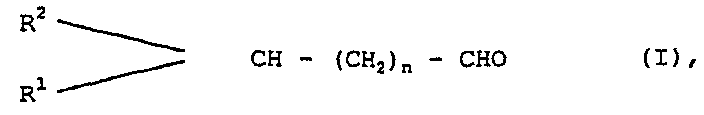 in which:
R¹, R² are identical or different and represent hydrogen, alkyl having from 1 to 3 carbon atoms, or an alkylthio group having one or two carbon atoms,
n represents an integer from 0 to 4,
a water content of up to 2 wt.% and an organic, saturated acid that forms metal complexes and does not have antioxidising action, which compositions exhibit improved storage stability.

2. Compositions according to claim 1,
**characterised in that**
they contain acid in an amount of from 0.004 to 0.1 wt.%, based on the amount of alkanal.

3. Compositions according to claims 1 and 2,
**characterised in that**
they contain a hydroxydicarboxylic acid.

4. Compositions according to claim 3,
**characterised in that**
they contain tartaric acid in chiral or racemic form.

5. Compositions according to claims 1 and 2,
**characterised in that**
they contain aminotrismethylenephosphonic acid.

6. Compositions according to claims 1 to 5,
**characterised in that**
they additionally contain alkanolamines or trialkanolamines.

7. Compositions according to claim 6,
**characterised in that**
they contain a trialkanolamine, alkyl corresponding to a C₁- to C₃-alkyl radical.

8. Compositions according to claims 6 and 7,
**characterised in that**
they contain a trialkanolamine in an amount of from 20 to 80 wt.%, based on the organic acid.

9. Process for improving the storage stability of aliphatic alkanals of the general formula (I) having a water content > 300 ppm,
**characterised in that**
there is added thereto an organic, saturated acid that forms metal complexes and does not have antioxidising action, optionally in the form of an aqueous or alcoholic solution.

10. Process according to claim 9,
**characterised in that**
the acid is added in an amount of from 0.04 to 0.1 wt.%, based on the amount of alkanal.

11. Process according to claims 9 and 10,
**characterised in that**
a hydroxydicarboxylic acid is added.

12. Process according to claim 11,
**characterised in that**
tartaric acid in chiral or racemic form is added.

13. Process according to claims 9 and 10,
**characterised in that**
aminotrismethylenephosphonic acid is added.

14. Process according to claims 9 to 12,
**characterised in that**
an alkanolamine or trialkanolamine is additionally added.

15. Process according to claim 14,
**characterised in that**
trialkanolamines are used in an amount of from 20 to 80 wt.%, based on the organic acid according to claim 1.

16. Process according to claims 14 and 15,
**characterised in that**
the alkanolamine or trialkanolamine is used in combination with from 0.005 to 0.015 wt.% of the acid, based on the alkanal.

## Revendications

1. Compositions contenant des alcanals aliphatiques de formule générale 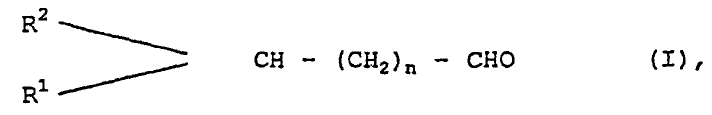 dans laquelle
R¹ et R² sont identiques ou différents et signifient un hydrogène, un alkyle comprenant 1 à 3 atomes de carbone ou une fois un groupe alkylthio comprenant un ou deux atomes de C,
n signifie un nombre entier de 0 à 4,
présentant une teneur en eau jusqu'à 2 % en poids et un acide organique saturé formant des complexes métalliques sans effet antioxydant, qui présentent une meilleure stabilité à l'entreposage.

2. Compositions selon la revendication 1, **caractérisées en ce qu'**elles contiennent de l'acide en une quantité de 0,004 à 0,1 % en poids par rapport à la quantité de l'alcanal.

3. Compositions selon les revendications 1 et 2, **caractérisées en ce qu'**elles contiennent un acide hydroxydicarboxylique.

4. Compositions selon la revendication 3, **caractérisées en ce qu'**elles contiennent de l'acide tartrique sous forme chirale ou racémique.

5. Compositions selon les revendications 1 et 2, **caractérisées en ce qu'**elles contiennent de l'acide aminotrisméthylènephosphonique.

6. Compositions selon les revendications 1 à 5, **caractérisées en ce qu'**elles contiennent en outre des alcanolamines ou des trialcanolamines.

7. Compositions selon la revendication 6, **caractérisées en ce qu'**elles contiennent une trialcanolamine, alkyle correspondant à un radical alkyle en C₁ à C₃.

8. Compositions selon les revendications 6 et 7, **caractérisées en ce qu'**elles contiennent une trialcanolamine en une quantité de 20 à 80 % en poids par rapport à l'acide organique.

9. Procédé pour améliorer la stabilité à l'entreposage d'alcanals aliphatiques de formule générale I, présentant une teneur en eau > 300 ppm, **caractérisé en ce qu'**on ajoute à ceux-ci un acide organique saturé formant des complexes métalliques sans effet antioxydant, le cas échéant sous forme d'une solution aqueuse ou alcoolique.

10. Procédé selon la revendication 9, **caractérisé en ce qu'**on ajoute l'acide en une quantité de 0,04 à 0,1 % en poids, par rapport à la quantité de l'alcanal.

11. Procédé selon les revendications 9 et 10, **caractérisé en ce qu'**on ajoute un acide hydroxydicarboxylique.

12. Procédé selon la revendication 11, **caractérisé en ce qu'**on ajoute de l'acide tartrique sous forme chirale ou racémique.

13. Procédé selon les revendications 9 et 10, **caractérisé en ce qu'**on ajoute de l'acide aminotrisméthylènephosphonique.

14. Procédé selon les revendications 9 à 12, **caractérisé en ce qu'**on ajoute en outre une alcanolamine ou une trialcanolamine.

15. Procédé selon la revendication 14, **caractérisé en ce qu'**on ajoute des trialcanolamines en une quantité de 20 à 80 % en poids par rapport à l'acide organique selon la revendication 1.

16. Procédé selon les revendications 14 et 15, **caractérisé en ce qu'**on utilise l'alcanolamine ou la trialcanolamine en combinaison avec 0,005 à 0,015 % en poids de l'acide par rapport à l'alcanal.
